# EUROPEAN PATENT APPLICATION

(11) **EP 4 585 255 A1**
(43) Date of publication of application: **16.07.2025**
(21) Application number: 24151149.2
(22) Date of filing: 10.01.2024
(51) Int. Cl.: A61N 1/05, A61N 1/375, A61N 1/362

(54) **IMPLANTABLE CARDIAC STIMULATION DEVICE AND METHOD FOR IMPLANTATION INTO A VENTRICLE IN A HEART AND FOR STIMULATING THE HEART WITH A LEFT BUNDLE BRANCH AREA PACING**

(71) Applicant: BIOTRONIK SE & Co. KG, 12359 Berlin (DE)
(72) Inventor: Dörr, Thomas, 12437 Berlin (DE)
(74) Representative: Biotronik Corporate Services SE

(57) **Abstract**

An implantable cardiac stimulation device (1) and a method for implanting same are presented. The device is configured for implantation into a ventricle (53) in a heart (51) and for stimulating the heart with a left bundle branch area pacing. It comprises:
an electronic pacing circuitry (3) configured for generating electric ventricle pulses for pacing ventricle actions in the heart;
an energy source (5) for providing energy to the electronic pacing circuitry;
a housing (7) accommodating the electronic pacing circuitry and the energy source; and
an elongate electrode assembly (9) being electrically connected to the electronic pacing circuitry, extending with a protruding portion (13) beyond the housing and including an electrical conductor (11) for transmitting the electric ventricle pulses to a tip (33) at the protruding portion of the electrode assembly.

The protruding portion of the electrode assembly comprises a proximal part (15) close to the housing and a distal part (17) distant from the housing, the proximal part being enclosed by an electrical insulation (19) and the distal part being electrically exposed. The protruding portion of the electrode assembly has a length (Lpp) of at least 5 mm and may be bendable and/or comprise an articulated joint.

## Description

The present invention relates to an implantable cardiac stimulation device for pacing a heart. Furthermore, the invention relates to a method for implanting such cardiac stimulation device.

Cardiac stimulation devices such as pacemakers are used for supporting a cardiac activity by providing electrical pulses to cardiac tissue in a patient's heart.

Conventionally, pacemakers have been implanted subcutaneously and one or more elongate lead electrodes have been introduced from a pacemaker housing into one or more of the cardiac chambers in the heart.

In a more modern approach, a miniaturized pacemaker may be implanted directly into one of the cardiac chambers. Such miniaturized pacemaker may also be referred to as implantable leadless pacemaker (iLP) or intracardiac pacemakers as its generally does not comprise any long elongate lead electrode but, instead, comprises an electrode being arranged directly at an outside surface of a housing of such miniaturized pacemaker. Due to space limitations, such iLP is generally implanted in a larger one of the cardiac chambers, i.e. in a ventricle of the heart. Furthermore, due to accessibility limitations, the iLP is generally implanted in the right ventricle of the heart which is easily accessible by introducing e.g. an implantation catheter along vessels of the patient. Therein, the housing of the iLP is typically anchored in cardiac tissue defining the right ventricle and the electrode of the iLP is in electrical contact with right ventricular cardiac tissue. Accordingly, electrical pulses generated by the iLP stimulate the heart at such right ventricular cardiac tissue.

However, while it is known that a natural activity of the heart is triggered via two bundle branches, i.e. via a right bundle branch and a left bundle branch, it has been observed that stimulating a heart by applying external electric pulses to its right ventricular cardiac tissue mainly simulates triggering the heart activity predominantly via the right bundle branch.

In fact, it has been found that, at least in some medical cases or in specific medical conditions, it may be beneficial to stimulate the heart by applying external electric pulses in a manner such as to simulate triggering the heart activity predominantly via the left bundle branch, such stimulation also being referred to as left bundle branch area pacing (LBBAP).

Accordingly, there may be a need for an implantable cardiac stimulation device and for a method for implanting such device beneficially enabling left bundle branch area pacing. Particularly, there may be a need for enabling a stimulation of the left bundle branch in a heart with a cardiac stimulation device and/or an implantation method enabling relatively simple, reliable and long-term stable implantation and cardiac pacing in a manner which, due to mainly the left bundle branch being paced, is gentle and corresponds well to natural stimulation activity within the heart.

Such needs may be met with the subject-matter of the independent claims. Advantageous embodiments are defined in the dependent claims as well as in the corresponding specification and figures.

According to a first aspect of the present invention, an implantable cardiac stimulation device is presented, the device being configured for implantation into a ventricle in a heart and for stimulating the heart with a left bundle branch area pacing. The device comprises (i) an electronic pacing circuitry configured for generating electric ventricle pulses for pacing ventricle actions in the heart, (ii) an energy source for providing energy to the electronic pacing circuitry, (iii) a housing accommodating the electronic pacing circuitry and the energy source and (iv) an elongate electrode assembly being electrically connected to the electronic pacing circuitry, extending with a protruding portion beyond the housing and including an electrical conductor for transmitting the electric ventricle pulses to a tip at the protruding portion of the electrode assembly. Therein, the protruding portion of the electrode assembly comprises a proximal part close to the housing and a distal part distant from the housing, the proximal part being enclosed by an electrical insulation and the distal part being electrically exposed. Particularly, the protruding portion of the electrode assembly has a length of at least 5 mm.

Within the frame of the application the term proximal is to be understood as close or closer to the implanter/the user of the implantation device. The term distal is to be understood as far away or more far away from the implanter/the user of the implantation device.

According to a second aspect of the invention, a method is presented, the method being configured for implanting an implantable cardiac stimulation device according to an embodiment of the first aspect of the invention into a ventricle in a heart and for stimulating the heart with a left bundle branch area pacing. The method comprises at least the following method steps, preferably in the indicated order:
inserting the stimulation device into the ventricle, and
introducing the protruding portion of the electrode assembly into a septum defining the ventricle wherein the tip of the protruding portion is introduced into a depth of the septum of at least 4 mm.

Briefly summarized in a non-limiting manner, ideas underlying embodiments of the present invention may be interpreted as being based, inter alia, on the following observations and recognitions:
As indicated further above, left bundle branch area pacing has been found to be beneficial for stimulating cardiac activity. However, while it was possible to implant an electrode lead coming from a subcutaneous pacemaker to cardiac tissue at the left ventricle, it was, until before the present invention, not obvious how to use or adapt a cardiac stimulation device which is adapted for implantation directly into a chamber of the heart, i.e. a cardiac stimulation device corresponding to an implantable leadless pacemaker, for left bundle branch area pacing. Particularly, it has been observed that, using for example conventional catheter-based implantation techniques, it appears hardly possible to implant any iPL directly into the left ventricle due to accessibility limitations in such implantation techniques.

It has now been found that left bundle branch area pacing may be enabled using a cardiac stimulation device which is configured for being implanted into the right ventricle in a heart while having a specific electrode assembly which enables introducing at least a portion of such electrode assembly into a septum defining the right ventricle in a manner such that an exposed tip of the electrode assembly contacts or at least comes close to the left bundle branch extending at or close to the septum. Accordingly, it is proposed to provide the stimulation device with an elongate electrode assembly having a sufficient length of at least 5 mm, preferably at least 7 mm, such as to extend from a housing of the stimulation device being positioned within the right ventricle towards an area at or close to the septum in which area extends the left bundle branch. Furthermore, it is proposed to provide at least a protruding portion of the electrode assembly with a proximal part in which an electrical conductor of the electrode assembly is enclosed by an electrical insulation, whereas a distal part of the electrode assembly is electrically exposed. Due to such implementation of the protruding portion of the electrode assembly, electric pulses generated by an electronic pacing circuitry within the housing of the stimulation device are transmitted via the electrical conductor in the electrode assembly towards the exposed distal part, this distal part being arranged at or close to the left bundle branch, whereas the proximal part of the electrode assembly extending between the housing and the distal part is electrically isolated against its environment such that no electric pulses are applied in such intermediate area including the right bundle branch. Accordingly, upon being correctly implanted in the right ventricle with its electrode assembly being introduced deep into the septum, the stimulation device may generate electric pulses which predominantly stimulate the left bundle branch of the heart.

Furthermore, additionally to generally enabling left bundle branch area pacing, it has been found that, using a stimulation device similar to an iLP arrangement for introducing and at least partly penetrating the septum in the heart may require arranging the housing of the stimulation device in a manner such as to prevent such housing from disturbing any pumping action of the heart. Particularly, while the electrode assembly should be introduced into the septum in a direction preferably being perpendicular to a septum surface, the housing should preferably be arranged within the ventricle in a direction not being perpendicular to such septum surface but preferably being parallel to such septum surface. Accordingly, as described in further detail below, embodiments of the stimulation device should be provided with an electrode assembly allowing, on the one hand, perpendicular introduction of the electrode assembly into the septum while, on the other hand, enabling approximately parallel arrangement of the housing with respect to the septum. Particularly, measures may be implemented such that the housing of the stimulation device may be arranged within the ventricle in a manner such as to avoid any unintended displacements of the housing or even almost free floating of the housing within the ventricle.

In the following, characteristics of embodiments of the present invention will be described in more detail.

The stimulation device presented herein is specifically configured for an implantation into a ventricle of a heart. For such purpose, the device needs to be sufficiently miniaturized, i.e. dimensions of its housing should be small enough for an accommodation within a volume available in the ventricle. Furthermore, materials used in portions or components of the device coming into direct contact with organic substances such as tissue or blood should be suitably biocompatible.

Furthermore, the stimulation device is configured for stimulating the heart with LBBAP. For such purpose, on the one hand, a geometry of components of the device is adapted such that electrical pulses may be applied predominantly to a cardiac tissue area adjacent to the left bundle branch. On the other hand, the electric pulses are configured e.g. with regards to their intensity, amplitude, timing, etc. for suitably stimulating the left bundle branch adjacent to the ventricle, such pulses therefore being referred to herein as "electric ventricle pulses".

The electronic pacing circuitry comprised in the stimulation device is specifically configured for generating such electric ventricle pulses. Specifically, the pacing circuitry may generate electric pulses regularly, preferably periodically. An intensity, an amplitude and/or a frequency of the pulses may be preset, may be tunable and/or may be adaptable to specific patient requirements and/or physiological conditions.

The energy source may store sufficient energy for a power supply of the electronic pacing circuitry for an extended lifetime of for example several years of operation. The energy source may store energy chemically and/or electrically. The energy source may for example be a battery or a capacitor. The energy source may be rechargeable and/or replaceable.

The housing may tightly and hermetically enclose the electronic pacing circuitry as well as the energy source. The housing may be made with or covered with biocompatible material. The housing may have outer dimensions of less than e.g. 8 cm, preferably less than 6 cm or less than 4 cm in length, i.e. in a longitudinal direction of the housing, and less than 2 cm, preferably less than 1.5 cm or less than 1 cm in width or diameter.

The electrode assembly includes an electrical conductor such as a metallic wire which, at its proximal end, is electrically connected to the electronic pacing circuitry and which extends to a distal end at a tip of the electrode assembly. Therein, the electrode assembly comprises an internal portion which extends within the housing, i.e. from the electronic pacing circuitry to a wall of the housing. Furthermore, the electrode assembly comprises a protruding portion which extends from the internal portion through a sealed opening in the housing to a tip being arranged outside the housing. Accordingly, the protruding portion protrudes beyond the housing.

A part of such protruding portion being closer to the housing than to the tip of the electrode assembly and therefore being referred to herein as proximal part is electrically enclosed by an electrical insulation. Such electrical insulation may be formed e.g. by a sheath, a sleeve or a covering layer of electrically insulating material such as polymer material. Another part of the protruding portion being closer to the tip of the electrode assembly than to the housing and therefore being referred to herein as distal part is electrically exposed, i.e. is not covered by any insulating material and may therefore come into direct electrical contact with environmental materials such as cardiac tissue.

Particularly, the protruding portion of the electrode assembly shall have a substantial length of at least 5 mm, preferably at least 7 mm, but preferably less than 80 mm, less than 50 mm, less than 30 mm or less than 15 mm. Accordingly, upon being introduced into the septum of the heart, the protruding portion may extend through an area of the septum adjacent to a right bundle branch and may reach with its tip to an area of the septum adjacent to the left bundle branch. Accordingly, as the tip of the protruding portion of the electrode assembly is exposed, electric ventricle pulses transmitted via the electrical conductor in the electrode assembly may effectively stimulate the left bundle branch.

Particularly, according to an embodiment, the proximal part of protruding portion of the electrode assembly may have a length of at least 3 mm.

In other words, the electrically insulated proximal part of protruding portion of the electrode assembly should be at least 3 mm long, preferably at least 4 mm long or at least 6 mm long, but usually less than 30 mm long or less than 15 mm long. Accordingly, at least 80% of the length of the protruding portion may be enclosed by the electrical insulation whereas only a minor fraction of such length at the tip end of the protruding portion is provided with the electrical conductor being exposed. Due to such configuration, the electrode assembly may be implanted with its proximal part of the protruding portion extending through a right side portion of the septum adjacent to the right ventricle accommodating the housing of the stimulation device while the distal part of the protruding portion reaching a left side portion of the septum adjacent to the left ventricle. As the right side portion of the septum includes or is close to the right bundle branch but the proximal part of the protruding portion of the electrode assembly is isolated at such location, the right bundle branch is not stimulated by any electrical ventricle pulses. In contrast hereto, as the left side portion of the septum includes or is close to the left bundle branch and the distal part of the protruding portion of the electrode assembly is exposed at such location, the left bundle branch is predominantly stimulated by the electrical ventricle pulses generated by the cardiac stimulation device.

According to an embodiment, the housing has an elongate shape with a length being at least twice as large as a width and the electrode assembly extends from a front face of the housing.

A housing having such elongate shape may enclose an inner volume which is sufficiently large for accommodating the electronic pacing circuitry and the energy source while still having a relatively small width or diameter. For example, the width or diameter may be substantially smaller than typical diameters of vessels of a patient, i.e. preferably smaller than 1.5 mm or smaller than 1 cm. Accordingly, during an implantation procedure, such housing may be transferred through a catheter having a relatively small diameter such that such catheter may be guided along vessels and into the heart chambers of a patient without excessively contacting or even dilating such vessels or heart chambers.

At such elongate housing, the electrode assembly may protrude from an end face, i.e. a distal face of the housing having a smaller area than a lateral area of the housing. In such configuration, during the implantation procedure, the housing may slide along an implantation catheter until the protruding portion of the electrode assembly at its distal front face is at least partly ejected from the catheter. In such stage, the distal end of the catheter may be arranged such that the ejected protruding portion of the electrode assembly is directed approximately perpendicular to the septum defining the right ventricle and may therefore be introduced into the septum at a right angle until its distal tip end reaches deep into the septum tissue.

However, in a case where the electrode assembly would be rigidly connected to the housing and would therefore rigidly protrude in a same direction as the housing, the housing would necessarily have to be arranged in line with the linear rigid electrode assembly. Therefore, in such case, the housing would be arranged such as to cross the ventricle, i.e. such as to extend at a right angle from a surface of the septum. However, in such crossing orientation, the elongate housing could hinder the heart from correctly deforming during the heart's pumping action as, in a contracted stage during the pumping action, the housing crossing the ventricle could come into mechanical contact with both, the septum at its one front face and the wall of the ventricle opposing the septum at its opposing rear face. In other words, the elongate housing being arranged in a crossing direction within the ventricle could interfere with the normal pumping motion within the ventricle.

In order to avoid such negative interference between the housing and the accommodating ventricle, according to an embodiment, the protruding portion of the electrode assembly may be configured to be bent by at least 60°, preferably at least 70°, at least 80° or at least 85°, but preferably less than 120°. Having such bending capacity in its electrode assembly, the stimulation device may be implanted according to an embodiment of the method proposed herein with a distal end portion of the protruding portion being introduced into the septum in a first direction, wherein a proximal end portion of the protruding portion together with the housing are arranged in a second direction being at an angle of at least 60° to the first direction.

In other words, the elongate protruding portion of the electrode assembly may be configured such as not to extend rigidly along a straight line but such as to enable obtaining a curved and/or angled geometry.

Particularly, the protruding portion may initially be provided in a straight configuration extending linearly in a direction along or parallel to a longitudinal direction of the housing of the stimulation device. In such initial configuration, the entire stimulation device including its electrode assembly may be accommodated within and is transferred along an elongate implantation catheter until reaching an intended implantation site within a right ventricle of a patient's heart. Upon arriving at such site, the protruding portion of the electrode assembly may be introduced into the septum at an approximately right angle to the septum surface such that, initially, also the implantation catheter and the stimulation device's housing are arranged at the approximately right angle to the septum surface adjoining the right ventricle. Upon having introduced the distal part of the electrode assembly in such manner, the housing of the stimulation device may then be finally ejected from the implantation catheter. Subsequently, the housing and/or the proximal part of the protruding portion of the electrode assembly may be subjected to a force in order to bend or angle at least a partial area of the protruding portion of the electrode assembly. Due to such bending or angling action, the housing may subsequently be arranged in a second direction differing from the first direction of the implanted distal end portion of the protruding portion of the electrode assembly by at least 60°, preferably by 90° ± 20°. Therein, the bending action may at least partially result from plastic deformation of at least partial areas of the protruding portion.

According to an embodiment, the protruding portion of the electrode assembly has a higher bending capacity in a proximal extension portion close to the housing than in a distal extension portion close to the tip.

Expressed differently, the protruding portion comprises a stiffer portion which is arranged close to the distal tip of the electrode assembly and which is therefore referred to as distal extension portion. Furthermore, the protruding portion comprises a more bendable portion which is arranged at the protruding portion in an area closer to the housing and which is therefore referred to as proximal extension portion. Such more bendable proximal extension portion has a lower stiffness than the stiffer portion and may therefore be bent more easily than the distal extension portion upon the protruding portion being subjected to any bending forces. Therein, the bending action may at least partially result from plastic deformation of the proximal extension portion.

Particularly, upon the protruding portion of the electrode assembly being introduced with its distal extension portion into septum tissue at the heart's septum, the bending action may preferably be effected at the proximal extension portion extending between the distal extension portion and the housing and being not introduced into the septum tissue. Accordingly, while the distal extension portion may extend linearly throughout the septum tissue, the proximal extension portion may be curved such as to allow the housing to be arranged at a non-rectangular angle with respect to the septum surface, i.e. at an angle of preferably more than 60° with respect to the distal extension portion.

According to an embodiment, the protruding portion comprises an articulated joint.

In other words, the protruding portion of the electrode assembly may be composed of at least two separate extension portions which are interconnected with each other via an articulated joint. Accordingly, a first extension portion for example closer to the housing may be arranged at an angle with respect to a second extension portion closer to a tip of the electrode assembly by angling the articulated joint in between both extension portions.

Using characteristics of the stimulation device as described herein before, according to an embodiment, the protruding portion of the electrode assembly may be configured to be deflected by at least one of plastic deformation and angling into a configuration in which a distal end portion of the protruding portion close to the tip extends in a first direction and a proximal end portion of the protruding portion close to the housing extends in a second direction being at an angle of at least 60° to the first direction.

Therein, it may be important to enable the deflection of the protruding portion of the electrode assembly in a permanently stable manner, i.e. by for example plastic deformation of at least a partial area of the protruding portion and/or by angling an articulated joint comprised in the protruding portion, such that the distal end portion of the electrode assembly may be arranged at an intended angle with respect to the proximal end portion of the electrode assembly, thereby also holding the housing, which is attached to such proximal end portion, at such intended angle. Accordingly, due to a mechanical stability of the protruding portion of the electrode assembly being partially fixed within the septum, the arrangement of and orientation of the housing is held in a stable configuration relative to the ventricle defined by such septum. Particularly, with the protruding portion being fixed with its distal end portion in the tissue of the septum, the housing being interconnected to such protruding portion is stably held within the ventricle at an intended location and with an intended orientation such as to prevent hindering any ventricle pumping motion.

Particularly, according to a further specified embodiment, an elasticity characteristics of the protruding portion of the electrode assembly and an interconnection characteristics of the proximal end portion of the protruding portion and of the housing may be configured such that, upon the distal end portion of the protruding portion being arranged in the first direction, the housing is elastically held by the proximal end portion of the protruding portion in a predetermined arrangement and orientation relative to the distal end portion.

In other words, while the shape and extension of the protruding portion of the electrode may intentionally be deflected by plastically deforming areas of the protruding portion and/or by angling an articulated joint, the protruding portion may additionally exhibit elasticity characteristics and the housing may be interconnected to the protruding portion in a manner such that the housing is not rigidly connected to the distal end portion of the protruding portion being fixed within the septum tissue but may at least slightly elastically move relative to such distal end portion.

Accordingly, the housing and the distal end portion of the electrode assembly may elastically displace relative to each other. Particularly, by suitably bending the electrode assembly and/or angling the articulated joint, the housing may be specifically arranged such that it becomes positioned at an intended location within the ventricle, for example such as to abut to a side wall or the septum defining the ventricle. Accordingly, the housing's positioning within the ventricle may be supported by such mechanical contact with the inner surface of the ventricle.

However, in order to avoid any negative interference of the housing with the ventricle, the inner surface of which continuously deflects during a pumping motion of the heart, a sufficient elasticity shall be established between the housing and the distal end portion of the electrode assembly introduced into the septum. As a result, at least a fraction of the forces generated for example by the weight of the housing may be supported by the mechanical contact of the housing with the ventricle's inner surface, thereby avoiding excessive forces to be exerted via the distal end portion of the electrode assembly onto the septum, while still allowing the housing to elastically displace relative to the distal end portion in order to follow the heart's pumping motion without hindering it.

According to an embodiment, the stimulation device may comprise an anchoring arrangement arranged at a rear side of the housing opposite to a front side of the housing at which the protruding portion extends beyond the housing, the anchoring arrangement being configured for anchoring the housing with respect to a trabecular meshwork within the ventricle. Thus, in accordance with an embodiment of the method proposed herein, after having introduced the protruding portion of the electrode assembly into the septum, at least one of the housing and the anchoring arrangement arranged at a rear side of the housing may be pushed into a configuration such as to contact ventricle wall tissue.

Expressed differently, the housing of the stimulation device may be provided with a specific arrangement at its rear side, such arrangement being configured for anchoring the housing within the ventricle, thereby preventing excessive displacements of the housing relative to inner surfaces of the ventricle for example upon any ventricle pumping motion. Particularly, such anchoring arrangement may be configured for interacting with the trabecular meshwork typically being present at the inner ventricle surface at a lower end of the ventricle at which the housing is to be accommodated. Accordingly, upon implanting the stimulation device, after having introduced the distal end portion of the electrode assembly into the septum, the housing may be specifically pushed into a configuration in which it abuts to tissue at an adjacent ventricle wall and/or in which its anchoring arrangement becomes anchored within the ventricle's trabecular meshwork.

According to a further specified embodiment, the anchoring arrangement may be configured for being elastically deployable between a retracted configuration with minimum lateral extension and an expanded configuration with maximum lateral extension.

Thus, the anchoring arrangement may initially be provided in its retracted configuration requiring a minimum space upon for example being included in an implantation catheter. In such configuration, the entire stimulation device together with its anchoring arrangement may be displaced within the implantation catheter. However, upon the stimulation device having been implanted at an intended site, its housing may be fully ejected from the implantation catheter and its anchoring arrangement may elastically deploy into its expanded configuration. In such expanded configuration, the anchoring arrangement extends along a maximum lateral extension such as to anchor for example at or within ventricle tissue structures such as the trabecular meshwork.

For example, the anchoring arrangement may comprise or consist of an elastic material such as silicone. The anchoring arrangement may comprise one or more elongate arms which may deploy relative to each other and/or relative to the housing from the retracted configuration to the expanded configuration, for example upon compression forces exerted by the implantation catheter being released.

According to an embodiment, the tip at the protruding portion of the electrode assembly may comprise a screw-like shape. In accordance with an embodiment of the implantation method proposed herein, the tip at the protruding portion of the electrode assembly may then be introduced into the septum by screwing-in.

In other words, the electrode assembly may have a screw-like structure at its distal tip. Such structure may be similar to a corkscrew. Using such screw-like structure, the electrode assembly may be introduced into the septum tissue easily and reliably using a rotating motion. This means, the electrode assembly may be successively screwed into the septum by turning it around its longitudinal axis. Thereby, the distal end of the electrode assembly may be precisely introduced into the septum tissue up to an intended depth at which it may stimulate the left bundle branch. Therein, the introduction depth may be controlled precisely, inter-alia, by controlling the number of rotations used for screwing the electrode assembly tip into the septum tissue.

It shall be noted that possible features and advantages of embodiments of the invention are described herein with respect to various embodiments of a cardiac stimulation device or embodiments of a method for implanting such stimulation device. One skilled in the art will recognize that the features may be suitably transferred from one embodiment to another and features may be modified, adapted, combined and/or replaced, etc. in order to come to further embodiments of the invention.

In the following, advantageous embodiments of the invention will be described with reference to the enclosed drawings. However, neither the drawings nor the description shall be interpreted as limiting the invention.
- Fig. 1: shows an implantable cardiac stimulation device according to an embodiment of the present invention in a straight configuration.
- Fig. 2: shows an implantable cardiac stimulation device according to an embodiment of the present invention in a bent configuration.
- Fig. 3: shows an implantable cardiac stimulation device according to another embodiment of the present invention having an articulated joint in an angled configuration.
- Fig. 4: visualizes an initial stage of a method for implanting a cardiac stimulation device according to an embodiment of the present invention.
- Fig. 5: visualizes a final stage of a method for implanting a cardiac stimulation device according to an embodiment of the present invention.

The figures are only schematic and not to scale. Same reference signs refer to same or similar features.

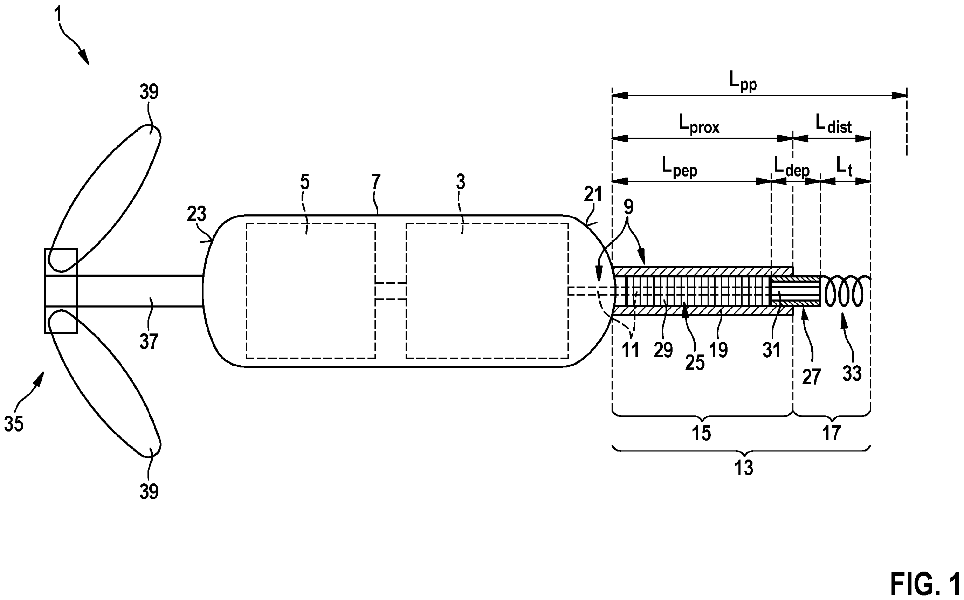

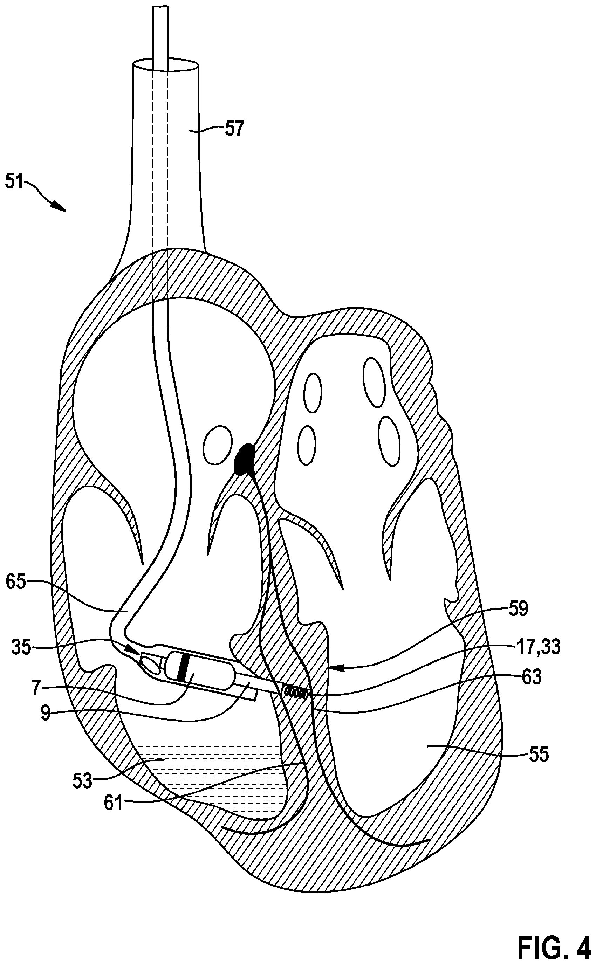

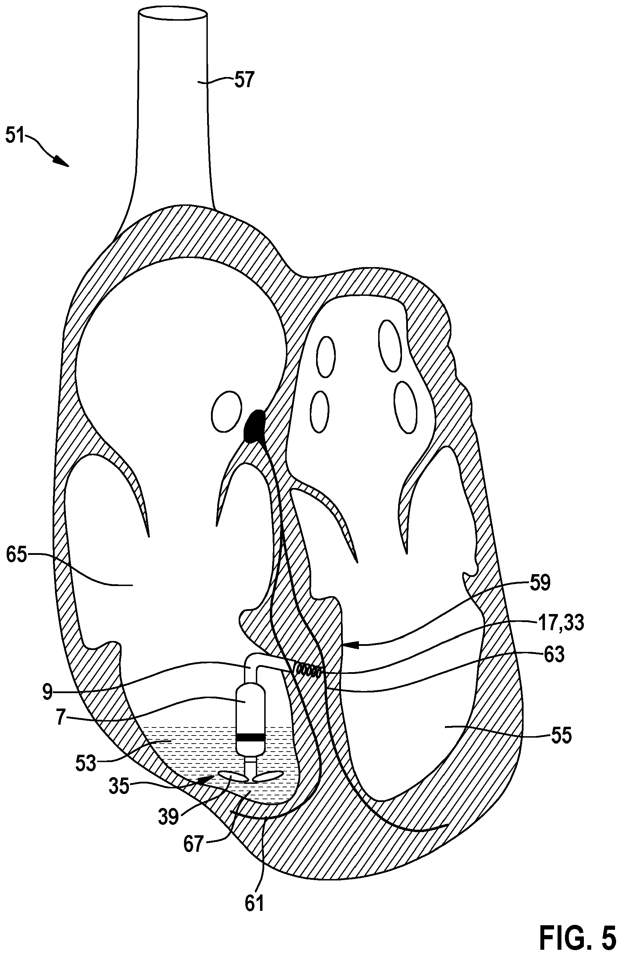

Fig. 1 shows an implantable cardiac stimulation device 1. Figs. 4 and 5 visualize the stimulation device 1 upon being implanted in a patient's heart 51. The device 1 is specifically configured for being implanted into a right ventricle 53 in the heart 51 and for stimulating the heart 51 with a pacing of a left bundle branch 63 within a septum 59.

The stimulation device 1 comprises an elongate housing 7 accommodating an electronic pacing circuitry 3 and an energy source 5. The pacing circuitry 3 may generate electric pulses for pacing ventricle actions in the heart 51. The energy source 5 may be for example a battery for providing electric power to the pacing circuitry 3.

The stimulation device furthermore comprises an electrode assembly 9 via which the electric pulses generated by the pacing circuitry 3 may be applied to cardiac tissue in the heart 51. Particularly, this electrode assembly 9 has an elongate shape with a substantial length of the electrode assembly 9 protruding beyond the housing 7. The electrode assembly 9 comprises an electric conductor 11 such as for example a metallic wire or metal core for electrically connecting the pacing circuitry 3 with a distal tip 33 of the electrode assembly 9. Specifically, an internal portion of the electrode assembly 9 extends within the housing 7 and is connected to the pacing circuitry 3, whereas an external portion of the electrode assembly 9 extends as a protruding portion 13 externally beyond the housing 7 at a front face 21 of the housing 7. This protruding portion 13 has an overall length Lpp of at least 5 mm, preferably more than 15 mm. Due to such significant length, the protruding portion 13 of the electrode assembly 9 or at least a fraction thereof may be introduced into the septum 59 down to a depth where its distal tip 33 is arranged closer to the left bundle branch 63 than to the right bundle branch 61.

Specifically, the protruding portion 13 of the electrode assembly 9 comprises a proximal part 15 close to the housing 7 as well as a distal part 17 distant from the housing 7. Therein, the proximal part 15 is enclosed by an insulation 19 for electrically insulating the electrical conductor 11 against a surrounding environment. In contrast hereto, the distal part 17 is not enclosed by such insulation 19 but is exposed such that electric pulses generated by the pacing circuitry 3 may be transmitted via the conductor 11 to the distal part 17 of the electrode assembly 9 at the distal tip 33, in order to thereby allow electrically stimulating cardiac tissue contacting the distal part 17. The proximal part 15 has a length Lprox of typically between 3 mm and 25 mm, such length corresponding to or being longer than a thickness of a part of the septum 59 including the right bundle branch 61. Accordingly, no electric pulses are transmitted to the right bundle branch 61 due to the electrical insulation 19 at the proximal part 15. In contrast hereto, the distal part 17, which may generally have a shorter length Ldist than the proximal part 15, may be arranged at a location within the septum 59 which is at or close to the left bundle branch 63.

In the example shown in Fig. 1, the protruding portion 13 comprises a proximal extension portion 25 being arranged close to the housing 7 and a distal extension portion 27 being arranged distant to such housing 7, i.e. close to the tip 33 of the electrode assembly 9. In the proximal extension portion 25, the electrode assembly 9 has a higher bending capacity, i.e. a lower stiffness, then in the distal extension portion 27. Such higher bending capacity may result for example from the proximal extension portion 25 being provided with a bendable sheath 29, whereas the stiffer distal extension portion 27 may be provided with a rigid sheath 31.

Accordingly, the proximal extension portion 25 may be bent in small bending radii of for example equal to or less than a length Lpep of the proximal extension portion 25. For example, such length Lpep of the proximal extension portion 25 may be between 2 mm and 20 mm. Accordingly, the proximal extension portion 25 may be bent by at least 60°, preferably by approximately 90°. Such bending action may be induced with relatively low bending forces, wherein such bending forces may be applied for example during an implantation procedure by using a catheter for pushing the housing 7 in an intended direction or orientation. In contrast hereto, due to its higher stiffness, the distal extension portion 27 is not or hardly bent upon application of such bending forces. A length Ldep of the distal extension portion 27 together with a length Lt of the tip 33 forming the non-isolated exposed distal part 17 of the electrode assembly 9 are generally shorter than the length Lpep of the proximal extension portion 25. For example, the sum of both lengths Ldep and Lt may correspond to a depths of a partial layer of the septum 59 in which the left branch bundle 63 extends. Typically, such depths is a few millimetres, for example between 3 mm and 10 mm, depending on individual conditions within the heart 51.

Accordingly, due to the two different extension portions 25, 27 along the protruding portion 13, the electrode assembly 9 may preferably be bent in an area of the proximal extension portion 25, wherein, upon being implanted in the heart 51, such area may generally extend outside the septum 59, whereas the distal extension portion 27 may be introduced septum 59 and may extend linearly throughout the tissue of the septum 59.

The stimulation device 1 further comprises an anchoring arrangement 35 extending from a rear (proximal) face 23 of the housing 7. The anchoring arrangement is provided with a rear protrusion 37 at which two deployable arms 39 are arranged such as to be passively and elastically deployable between a retracted configuration with a minimum lateral extension and an expanded configuration (as shown in Fig. 1) with a maximum lateral extension. The arms 39 and the rear protrusion 37 may be provided as separate components, wherein the arms 39 may be hinged to the rear protrusion 37 and may be pretensioned towards their expanded configuration. Alternatively, the arms 39 and the rear protrusion 37 may be combined in a single integral component. The arms 39 and/or the rear protrusion 39 may comprise or consist of an elastic material such as silicone.

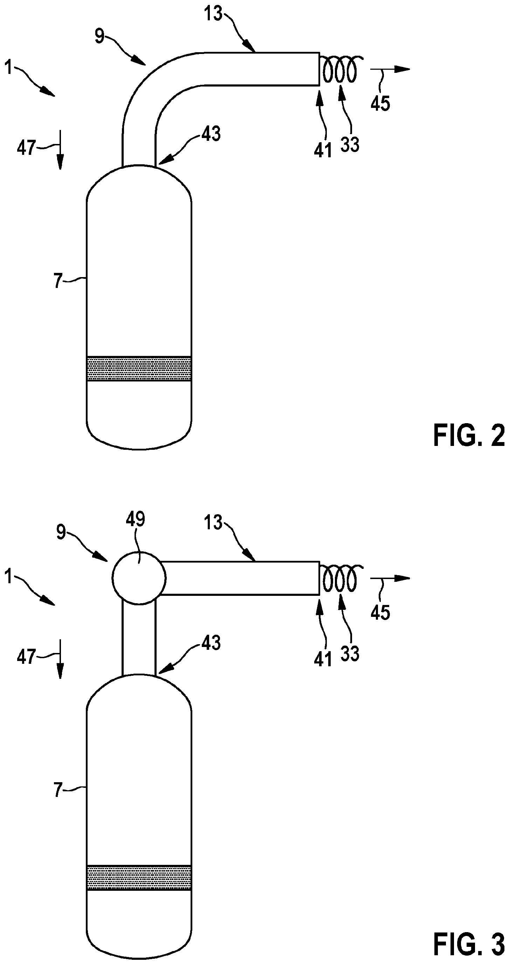

While Fig. 1 shows the stimulation device 1 with its electrode assembly 9 being in a straight configuration, Fig. 2 shows the electrode assembly 9 in a bent configuration. Therein, a distal end portion 41 of the protruding portion 13 close to the tip 33 extends in a first direction 45 (i.e. in the figure horizontally) whereas a proximal end portion 43 as well as the housing 7 extend in a second direction 47 (i.e. in the figure vertically) being at approximately a right angle relative to the first direction 45.

It may be noted that, instead of providing the protruding portion 13 of the electrode assembly 9 with two different extension portions, i.e. with the proximal extension portion 25 and the distal extension portion 27 differing with regards to their bending capacity, according to an alternative embodiment, the entire protruding portion 13 may be provided with a homogeneous bending capacity.

Fig. 3 represents another embodiment of the stimulation device 1. Therein, the protruding portion 13 of the electrode assembly 9 is provided with an articulated joint 49. Using this articulated joint 49, the distal end portion 41 and the proximal end portion 43 may extend in differing first and second directions 45, 47. The articulated joint 49 may act as a hinge between the housing 7 and the proximal end portion 43, on the one side, and the distal end portion 41 including the exposed tip 43, on the other side. The articulated joint 49 may be articulated between a straight configuration and an angled configuration with an articulation angle being for example between 0° and 120°. The articulated joint 49 may be elastically pretensioned towards its angled configuration.

It is to be noted that, for easier representation, Figs. 2 and 3 have been simplified and do not show some of the details presented in Fig. 1; for example, a representation of the anchoring arrangement 35 has been omitted.

Next, stages of a method for implanting the cardiac stimulation device 1 will be described with reference to Figs. 4 and 5.

In an initial stage, an implantation catheter 65 is introduced into a patient and is guided along vessels 57 until reaching the heart 51. There, the catheter 65 is led into the right ventricle 53. Specifically, a distal end of the catheter 65 is arranged at the destination site such as to be oriented in approximately a right angle with regards to the septum 59 separating the right ventricle 53 from the left ventricle 55. The stimulation device 1 is included inside the catheter 65.

Upon reaching the destination site, the stimulation device 1 is partly ejected from the catheter 65 such that at least its protruding portion 13 protrudes from the catheter 65. The catheter 65 together with the stimulation device 1 are then approached towards the septum 59 and, in order to introduce the electrode assembly 9 into the septum 59, the stimulation device 1 is slowly rotated such that its screw-shaped tip 33 at the distal end of the protruding portion 13 is precisely screwed into the tissue of the septum 59. Specifically, such introduction action is executed such that the exposed distal part 17 of the electrode assembly 9 reaches an area at or close to the left bundle branch 63, whereas the electrically insulated proximal part 15 of the electrode assembly 9 extends along the right bundle branch 61.

After having introduced the electrode assembly 9 into the septum 59 in such manner, the stimulation device 1 may be completely ejected from the catheter 65 by for example retracting the catheter 65. Subsequently, the housing 7 of the stimulation device 1 being released in such manner may be pushed in a downward direction using for example the catheter 65 or a specific pushing tool. Due to such pushing action, the bendable protruding portion 13 of the electrode assembly 9 may be plastically deformed into a curved configuration such that the housing 7 attached thereto no more extends in line with the distal extension portion 27 of the electrode assembly 9, i.e. in a substantially horizontal direction crossing the ventricle 53, but is arranged at an angle of at least 60° or at least 80° thereto. Accordingly, the elongate housing 7 is then arranged approximately in a vertical direction, such direction corresponding to a main extension direction of the ventricle 53. Upon being oriented in such manner, the housing 7 does no more hinder any contracting pumping motion of the ventricle 53.

Furthermore, the housing 7 may be pushed and deflected such that its anchoring arrangement and/or a distal end area of the housing itself are arranged such as to come into contact with ventricle wall tissue. Particularly, upon having been ejected from the catheter 65 in which it was stored in a retracted configuration, the anchoring arrangement 35 may have deployed to its extended configuration in which its arms 39 are deflected in an outward direction such as to obtain a maximum diameter. With such deflected anchoring arrangement 35, the housing 7 may be anchored for example at trabecular meshwork 67 at a lower area of the right ventricle 53. Additionally or alternatively, a portion of the housing 7 at or close to its rear surface 23 may be arranged such as to abut against some cardiac tissue at an inner surface of the ventricle 53. The anchoring and/or mechanical abutment as well as mechanical characteristics of the stimulation device 1 itself may be set such as to enable at least some elastic deformation between the anchoring arrangement 35 and/or the housing 7, on the one side, and the distal part 17 of the electrode assembly 9, on the other side, thereby allowing the implanted stimulation device 1 to follow some pumping heart motion to a sufficient degree. Due to such anchoring and/or mechanical abutment, the housing 7 may be mechanically stabilised within the ventricle 53 and excessive bending forces or displacement forces may be prevented from acting onto the electrode assembly 9 being introduced into the septum 59.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### List of Reference Numerals

1 cardiac stimulation device
3 electronic pacing circuitry
5 energy source
7 housing
9 electrode assembly
11 electrical conductor
13 protruding portion
15 proximal part
17 distal part
19 insulation
21 front face of the housing
23 rear face of the housing
25 proximal extension portion
27 distal extension portion
29 bendable sheath
31 rigid sheath
33 tip with screw-shape
35 anchoring arrangement
37 rear protrusion
39 deployable arm
41 distal end portion
43 proximal end portion
45 first direction
47 second direction
49 articulated joint
51 heart
53 right ventricle
55 left ventricle
57 vessel
59 septum
61 right bundle branch
63 left bundle branch
65 catheter
67 trabecular meshwork
Lpp length of protruding portion
Lprox length of proximal part
Ldist length of distal part
Lpep length of proximal extension portion
Ldep length of distal extension portion
Lt length of tip

## Claims

1. Implantable cardiac stimulation device (1) for implantation into a ventricle (53) in a heart (51) and for stimulating the heart (51) with a left bundle branch area pacing, the device (1) comprising:
an electronic pacing circuitry (3) configured for generating electric ventricle pulses for pacing ventricle actions in the heart (51);
an energy source (5) for providing energy to the electronic pacing circuitry (3);
a housing (7) accommodating the electronic pacing circuitry (3) and the energy source (5);
an elongate electrode assembly (9) being electrically connected to the electronic pacing circuitry (3), extending with a protruding portion (13) beyond the housing (7) and including an electrical conductor (11) for transmitting the electric ventricle pulses to a tip (33) at the protruding portion (13) of the electrode assembly (9),
wherein the protruding portion (13) of the electrode assembly (9) comprises a proximal part (15) close to the housing (7) and a distal part (17) distant from the housing (7),
the proximal part (15) being enclosed by an electrical insulation (19) and the distal part (17) being electrically exposed,
wherein the protruding portion (13) of the electrode assembly (9) has a length (Lpp) of at least 5 mm.

2. Implantable cardiac stimulation device (1) of claim 1,
wherein the proximal part (15) of protruding portion (13) of the electrode assembly (9) has a length (Lprox) of at least 3 mm.

3. Implantable cardiac stimulation device (1) of any one of the preceding claims,
wherein the housing (7) has an elongate shape with a length being at least twice as large as a width, and
wherein the electrode assembly (9) extends from a front face (21) of the housing (7).

4. Implantable cardiac stimulation device (1) of any one of the preceding claims, wherein the protruding portion (13) of the electrode assembly (9) is configured to be bent by at least 60°.

5. Implantable cardiac stimulation device (1) of any one of the preceding claims, wherein the protruding portion (13) of the electrode assembly (9) has a higher bending capacity in a proximal extension portion (25) close to the housing (7) than in a distal extension portion (27) close to the tip (33).

6. Implantable cardiac stimulation device (1) of any one of the preceding claims, wherein the protruding portion (13) comprises an articulated joint (49).

7. Implantable cardiac stimulation device (1) of any one of the preceding claims, wherein the protruding portion (13) of the electrode assembly (9) is configured to be deflected by at least one of plastic deformation and angling into a configuration in which a distal end portion (41) of the protruding portion (13) close to the tip (33) extends in a first direction (45) and a proximal end portion (43) of the protruding portion (13) close to the housing (7) extends in a second direction (47) being at an angle of at least 60° to the first direction (45).

8. Implantable cardiac stimulation device (1) of claim 7,
wherein an elasticity characteristics of the protruding portion (13) of the electrode assembly (9) and an interconnection characteristics of the proximal end portion (43) of the protruding portion (13) and of the housing (7) are configured such that, upon the distal end portion (41) of the protruding portion (13) being arranged in the first direction (45), the housing (7) is elastically held by the proximal end portion (43) of the protruding portion (13) in a predetermined arrangement and orientation relative to the distal end portion (41).

9. Implantable cardiac stimulation device (1) of any one of the preceding claims, further comprising
an anchoring arrangement (35) arranged at a rear face (23) of the housing (7) opposite to a front face (21) of the housing (7) at which the protruding portion (13) extends beyond the housing (7), the anchoring arrangement (35) being configured for anchoring the housing (7) with respect to a trabecular meshwork (67) within the ventricle (53).

10. Implantable cardiac stimulation device (1) of claim 9,
wherein the anchoring arrangement (35) is configured for being elastically deployable between a retracted configuration with minimum lateral extension and an expanded configuration with maximum lateral extension.

11. Implantable cardiac stimulation device (1) of any one of the preceding claims,
wherein the tip (33) at the protruding portion (13) of the electrode assembly (9) comprises a screw-like shape.

12. Method for implanting an implantable cardiac stimulation device (1) according to any one of the claims 1 to 11 into a ventricle (53) in a heart (51) and for stimulating the heart (51) with a left bundle branch area pacing, the method comprising:
inserting the stimulation device (1) into the ventricle (53), and
introducing the protruding portion (13) of the electrode assembly (9) into a septum (59) defining the ventricle (53), wherein the tip (33) of the protruding portion (13) is introduced into a depth of the septum (59) of at least 4 mm.

13. Method of claim 12,
wherein a distal end portion (41) of the protruding portion (13) is introduced into the septum (59) in a first direction (45) and wherein a proximal end portion (43) of the protruding portion (13) together with the housing (7) are arranged in a second direction (47) being at an angle of at least 60° to the first direction (45).

14. Method of any one of claims 12 and 13,
wherein, after having introduced the protruding portion (13) of the electrode assembly (9) into the septum (59), at least one of the housing (7) and an anchoring arrangement (35) arranged at a rear face of the housing (7) is pushed into a configuration such as to contact ventricle wall tissue.

15. Method of any one of claims 12 to 14,
wherein the tip (33) at the protruding portion (13) of the electrode assembly (9) is introduced into the septum (59) by screwing-in.
